# EUROPEAN PATENT SPECIFICATION

(11) **EP 2 221 015 B1**
(45) Date of publication and mention of the grant of the patent: **12.10.2011**
(21) Application number: 10154357.7
(22) Date of filing: 23.02.2010
(51) Int. Cl.: A61B 17/88

(54) **Expandable Counter-torque wrench**
Ausziehbarer Gegenwirkmoment-Schraubenschlüssel
Clé à couple de réaction extensible

(30) Priority: 24.02.2009 US 391461
(43) Date of publication of application: 25.08.2010
(73) Proprietor: Aesculap Implant Systems, LLC, Center Valley, PA 18034 (US)
(72) Inventor: Buss, Donald A., Macungie, PA 18062 (US); Weaver, Paul, Douglassville, PA 19518 (US); Love, John, Allentown, PA 18104 (US)
(74) Representative: Hoeger, Stellrecht & Partner Patentanwälte

(56) References cited:
- US-A1- 2006 111 712
- US-A1- 2006 260 440
- US-A1- 2008 172 062

## Description

### FIELD OF THE INVENTION

The present invention relates generally to an expandable counter-torque wrench for positioning and securing spinal fixation rods to screw and hook implants in accordance with the preamble of claim 1.

### BACKGROUND OF THE INVENTION

A number of pedicle screw systems in the state of the art include a screw or hook implant attached to a receiver body for receiving a spinal rod. The receiver body typically includes a channel for receiving and seating the rod. A locking element, such as a set screw, is inserted into the channel to lock the rod in place in the receiver body. The set screw is secured in place by rotating the set screw relative to the receiver body.

In order to keep the receiver body and rod from rotating as the set screw is being screwed to the receiver body, a counter-torque wrench may be placed around the receiver body to counter the torque generated as the set screw is screwed into the receiver body. In some instances, however, as the set screw is being screwed into the receiver body, the receiver body may splay open, causing the receiver body to jam or bind inside the counter-torque wrench, and making it difficult to remove the counter-torque wrench from the receiver body after the set screw has been inserted.

A spinal fixation tool set and method are known from US 2006/0111712 A1, from which the preamble of claim 1 departs. Moreover, a bone anchor manipulation device is disclosed in US 2008/0172062 A1.

In view of the foregoing, known counter-torque wrenches leave much to be desired in terms of ergonomics and functionality.

### SUMMARY OF THE INVENTION

Briefly, the deficiencies of the known prior art are overcome in accordance with the present invention by an expandable counter-torque wrench for positioning and securing spinal fixation rods to screw and hook implants in accordance with claim 1. Further preferred embodiments are defined in the dependent claims.

It is expedient if the tubular outer body includes an internal thread and wherein the inner shaft includes an external thread in cooperative engagement with the internal thread.

It is expedient if the inner shaft comprises a generally tubular body extending between the gripping end portion and the proximal end.

It is advantageous if the plurality of branches comprises an even number of branches.

It is expedient if the counter-torque wrench further comprises a torque-applying handle operatively connected to the tubular inner shaft.

It is advantageous if the outer body is rotatable relative to the inner shaft to move the inner shaft relative to the outer body between the relative closed position and the relatively open position.

It is expedient if the inner shaft further comprises a handle end portion, and wherein the torque-applying handle is fixedly coupled to the handle end portion.

It is advantageous if the inner shaft is cannulated, forming a bore extending between the gripping end portion and the handle end portion.

It is expedient if the outer body comprises a conically shaped inner surface.

It is advantageous if the gripping end portion of the inner shaft comprises a tapered outer surface disposed proximally of the branches.

It is expedient if the conically shaped inner surface comprises a nub extending radially inwardly in slidable engagement with the tapered outer surface on the inner shaft.

It is advantageous if the plurality of branches comprises a collet.

It is expedient if the plurality of branches comprises a pair of diametrically opposed cut outs for receiving a fixation rod.

### BRIEF DESCRIPTION OF THE DRAWINGS

The foregoing summary, as well as the following detailed description of preferred embodiments of the invention, will be better understood when read in conjunction with the appended drawings, which are incorporated herein and constitute part of this specification. For purposes of illustrating the invention, there are shown in the drawings exemplary embodiments of the present invention. It should be understood, however, that the invention is not limited to the precise arrangements and instrumentalities shown. In the drawings, the same reference numerals, are employed for designating the same elements throughout the several figures. In the drawings:
FIG. 1 is a perspective view of a counter-torque wrench according to an exemplary embodiment of the present invention, with the counter-torque wrench in a gripping position;
FIG. 2 is an enlarged view of the distal end of the counter-torque wrench of FIG. 1;
FIG. 3 is a perspective view of the counter-torque wrench of Fig. 1 in a release position;
FIG. 4 is an enlarged view of the distal end of the counter-torque wrench of FIG. 3;
FIG. 5 is a side elevational view of the counter-torque wrench of FIG. 1, with an implant affixed thereto;
FIG. 6 is a side elevational view of an outer body of the counter-torque wrench of FIG. 1;
FIG. 7 is a sectional view of the outer body of FIG. 6, taken along line 7--7 of Fig. 6;
FIG. 8 is an enlarged view of the distal end of the outer body shown in FIG. 7;
FIG. 9 is a side elevational view of an inner shaft of the counter-torque wrench of FIG. 1;
FIG. 10 is a sectional view of a distal end of the inner shaft of FIG. 9, taken along line 10--10 of FIG. 9;
FIG. 11 is an enlarged view of the distal end of the inner shaft shown in FIG. 10;
FIG. 12 is a sectional view of the inner shaft of FIG. 9, taken along line 12--12 of FIG. 9;
FIG. 13 is a side elevational view of a drive nut of the counter-torque wrench of FIG. 1;
FIG. 14 is a sectional view of the drive nut of FIG. 13, taken along line 14--14 of Fig. 13;
FIG. 15 is a sectional view of a portion of the counter-torque wrench of FIG. 1 including the outer body of FIG. 6, the inner shaft of FIG. 9, and the drive nut of FIG. 13;
FIG. 16 is an enlarged view of the proximal end of the assembly shown in FIG. 15;
FIG. 17 is an enlarged view of the distal end of the assembly shown in FIG. 15;
FIG. 18 is an enlarged view of an engagement point of the outer body and the inner shaft of FIG. 15;
FIG. 19 is a side elevational view of a handle of the counter-torque wrench of FIG. 1; and
FIG. 20 is a flowchart illustrating exemplary steps for operating the counter-torque wrench of the present invention.

### DETAILED DESCRIPTION OF EXEMPLARY EMBODIMENTS OF THE INVENTION

Certain terminology is used herein for convenience only and is not to be taken as a limitation on the present invention. The terminology includes the words specifically mentioned, derivatives thereof and words of similar import. As used herein, the term "proximal" is intended to define a direction closer to an operator of a counter-torque wrench according to the present invention, and the term "distal" is intended to define a direction closer to a patient on which the counter-torque wrench according to the present invention is used.

The following describes an exemplary embodiment of the present invention. It should be understood based on this disclosure, however, that the invention is not limited by this exemplary embodiment. A counter-torque wrench according to an exemplary embodiment of the present invention is used to provide a surgeon with a tool capable of constraining an implant with the added advantage of a release mechanism that aids in removing the counter-torque wrench from the implant after the implant has been inserted into the patient. An exemplary implant for which the present invention may be used is the Aesculap Implant Systems, Inc. S-4 5.5 millimeter rod implant, although those skilled in the art will recognize that other implant designs may be used with the counter-torque wrench of the present invention.

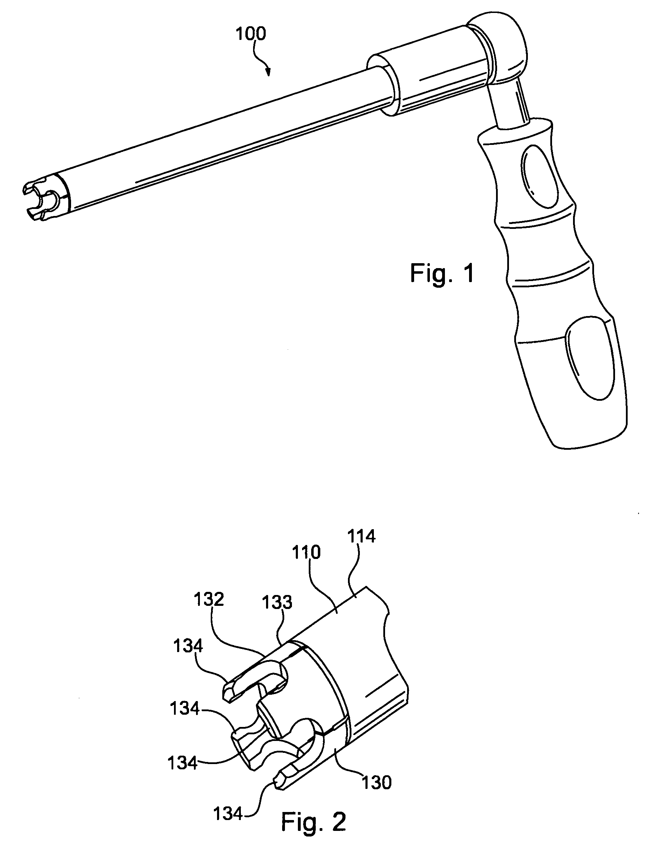

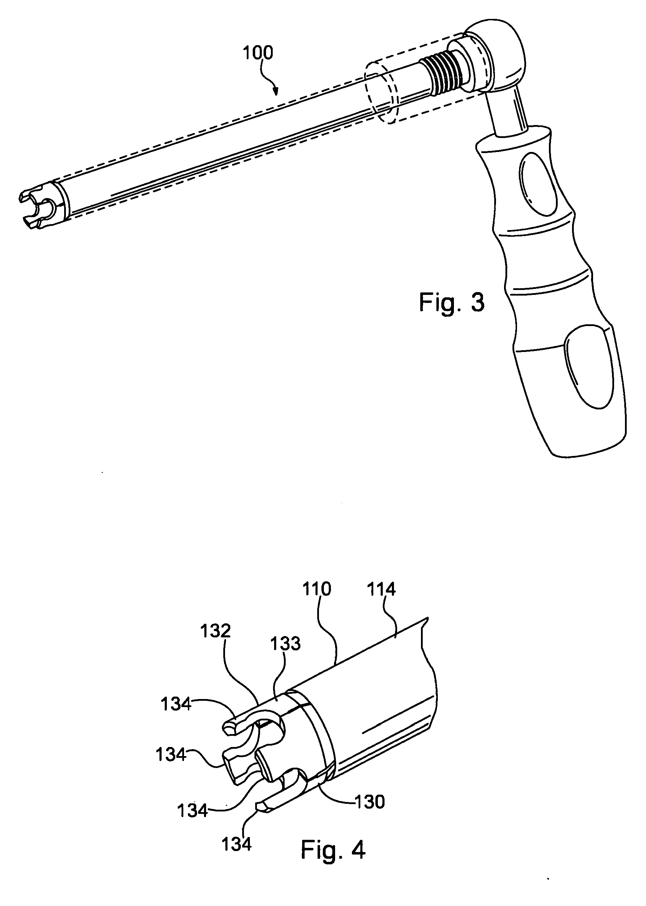

Referring to the drawing figures generally, a counter-torque wrench 100 in accordance with an exemplary embodiment of the present invention is shown. Counter-torque wrench 100 is operable between a closed or gripping position, as shown in FIGS. 1 and 2, and an open or release position, as shown in FIGS. 3 and 4.

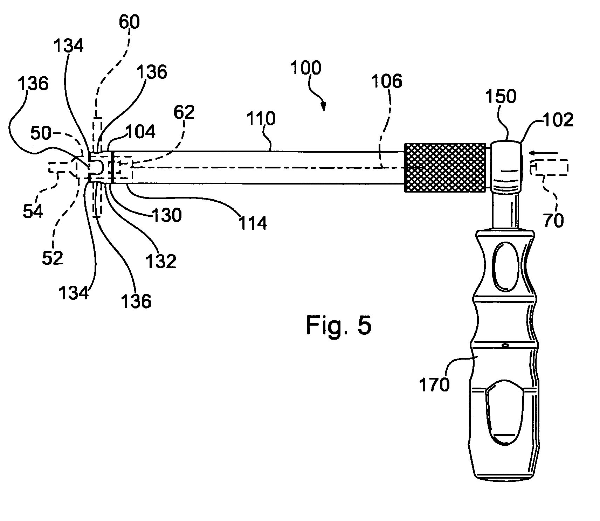

Counter-torque wrench 100 is used to stabilize the position of a rod implant as the rod is being secured in its rod receiver. Referring to FIG. 5, in which an exemplary implant 50 is shown affixed to counter-torque wrench 100, implant 50 includes a rod receiver in the form of a screw cap 52 through which a polyaxial screw 54 extends. Screw cap 52 includes a pair of diametrically opposed slots (not shown in FIG. 5) extending partially therethrough. A rod 60 is seated within the slots. A set screw 62 is inserted into screw cap 52 and is screwed into screw cap 52 by a screw driver 70 to secure rod 60 to screw cap 52.

In many circumstances, a surgeon must apply a significant amount of torque on set screw 62 to drive set screw 62 in screw cap 52 and advance rod 60 into a fully seated position. This torque is transferred from set screw 62 to screw cap 52. If screw cap 52 is not restrained, screw cap 52 and rod 60 will be allowed to rotate with set screw 62. Counter-torque wrench 100 is inserted over the proximal end of implant 50 such that counter-torque wrench 100 engages rod 60 to prevent rod 60 from rotating as set screw 62 is screwed into place.

Counter-torque wrench 100 includes a tubular outer body 110 and an inner shaft 130 co-axially disposed within outer body 110. Outer body 110 is axially translatable with respect to inner shaft 130 to move counter-torque wrench 100 between the gripping position of FIGS. 1 and 2 and the release position of FIGS. 3 and 4. Referring back to FIG. 5, a drive nut 150 is coupled to inner shaft 130 and a torque-applying handle 170 extends from drive nut 150.

Counter-torque wrench 100 includes a proximal end 102 and a distal end 104, with a linear axis 106 extending between proximal end 102 and distal end 104. Linear axis 106 extends through both outer body 110 and inner shaft 130 when inner shaft 130 is disposed within outer body 110, as is shown in FIG. 5. Handle 170 extends from proximal end 102, generally orthogonally relative to linear axis 106.

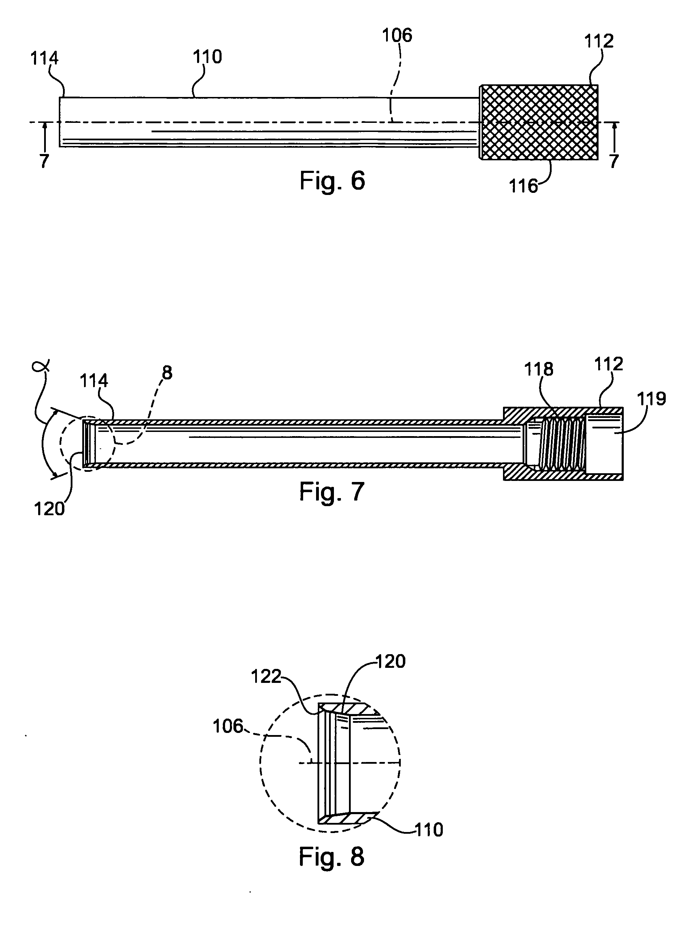

Referring now to FIGS. 6-8, outer body 110 includes a proximal end 112 and a distal end 114. Proximal end 112 of outer body 110 includes a knurled gripping portion 116. As shown in FIG. 6, knurled gripping portion 116 has a larger outer diameter than a remainder of outer body 110, although those skilled in the art will recognize that knurled gripping portion 116 may have an outer diameter approximately the same as the remainder of outer body 110.

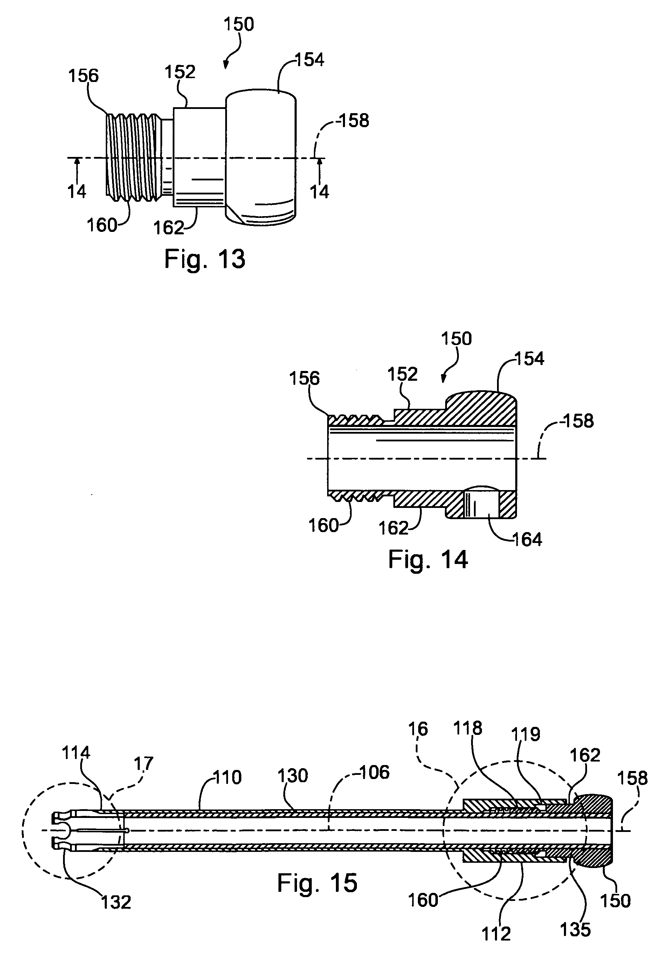

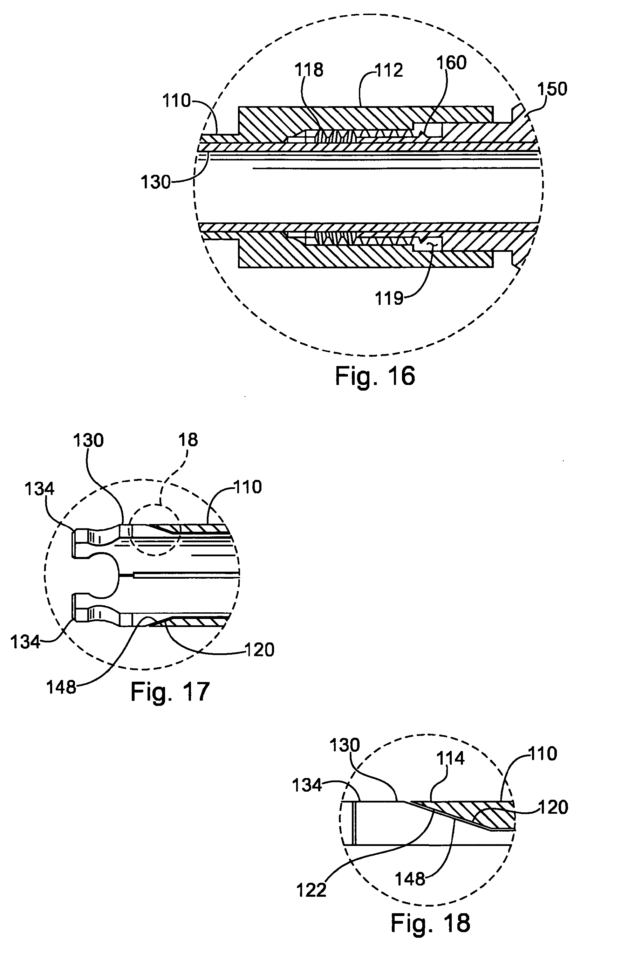

As shown in FIG. 7, proximal end 112 of outer body 110 also includes an internal thread 118. Internal thread engages inner shaft 130 (shown in FIGS. 15 and 16) and provides for axial translation of outer body 110 with respect to inner shaft 130. Proximal end 112 of outer body 110 further includes a recessed cylindrical bore 119 located proximal of internal thread 118. Bore 119 is slightly larger than the outer diameter of drive nut 150 where the nut contacts the bore to allow proximal end 112 of outer body 110 to slide along drive nut 150 (shown in FIGS. 15 and 16) when outer body 110 is translated relative to inner shaft 130.

Distal end 114 of outer body 110 includes a conically shaped inner surface 120. In an exemplary embodiment, inner surface 120 is angled at an included angle a of about 40 degrees. Referring to the enlargement of conically tapered surface 120 in FIG. 8, inner surface 120 comprises a radially extending nub 122 extending around the entire inner perimeter of inner surface 120 (*i.e.* 360 degrees), generally toward longitudinal axis 106. While an exemplary embodiment of counter-torque wrench 100 includes nub 122 extending 360 degrees around inner surface 120, those skilled in the art will recognize that nub 122 may extend less than 360 degrees around inner surface 120 and that a plurality of nubs 122 may be used.

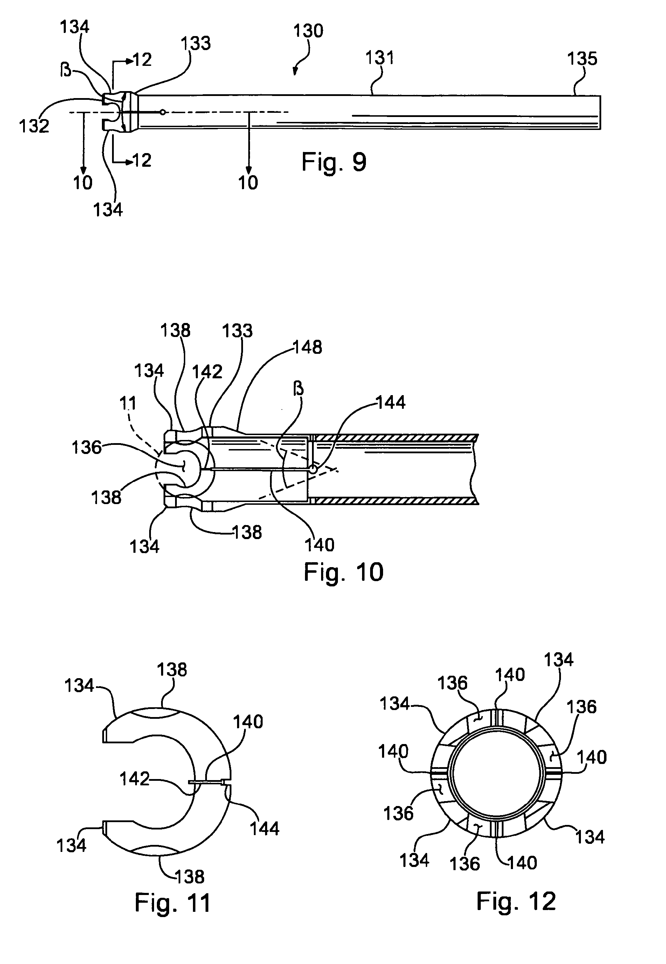

Referring now to FIGS. 9-12, inner shaft 130 includes a generally tubular body 131 that is sized to allow a driving tool, such as screw driver 70 (shown in FIG. 5), or other implement, to be inserted therethrough. Referring also to FIGS. 2 and 4, inner shaft 130 further includes a distal, or gripping end portion 132, disposed proximate to distal end 114 of outer body 110 and a proximal, or handle end, portion 135 (shown in FIG. 9). Inner shaft 130 is cannulated, forming a bore extending between gripping end portion 132 and handle end portion 135.

Gripping end portion 132 includes a collet 133 having a plurality of branches 134 extending distally of distal end 114 of outer body 110. Branches 134 are radially displaceable between a relatively open position, in which the branches are naturally displaced radially outwardly from one another (as shown in FIG. 4), and a biased, or closed position, in which the branches are displaced radially inwardly toward one another by outer body 110 to form a cylinder (as shown in FIG. 2). The distance between branches 134 is greater in the open position than in the closed position. In the embodiment shown, branches 134 extend away from longitudinal axis 106 at an angle of about 0.4 degrees in the open position. That is, the inner surface of each branch 134 tapers radially outwardly from longitudinal axis 106, from the proximal end of the branch to the distal end. In this arrangement, the inner surfaces of branches 134 form a cylindrical socket when the branches converge inwardly into the closed position. If desired, the angle of taper inside branches 134 can be adjusted so that the internal profile of the branches in the closed position assumes a tapered or conical shape to match an anticipated amount of radial splaying exhibited by a receiver body.

In the illustrated exemplary embodiment of counter-torque wrench 100, four branches 134 are used. While four branches 134 are used in the illustrated exemplary embodiment, those skilled in the art will recognize that two or possibly even six branches 134 could be used. In an exemplary embodiment, an even number of branches 134 is used so that rod 60 on implant 50 may be inserted within diametrically opposed cutouts 136 between adjacent branches 134, as shown in FIG. 5.

Each branch 134 includes an arcuate portion 138 formed along either side of branch 134. As shown in FIG. 10, arcuate portions 138 of two adjacent branches 134 form a cutout 136 between adjacent branches 134. Arcuate portions 138 are curved to approximately the same radius of curvature as rod 60 such that, when counter-torque wrench 100 is disposed over implant 50 as shown in FIG. 5, rod 60 is seated within cutouts 136 with linear contact between rod 60 and arcuate portions 138.

Adjacent branches 134 are separated from each other by a longitudinal gap 140. At a distal end portion 142, gap 140 may be about 0.1 millimeters across, while a length of gap 140 extending proximally of distal end portion 142 may be about 0.3 millimeters across. A proximal end 144 of gap 140 may be enlarged to a circle having a diameter of about 1 millimeter to provide stress relief to branches 134.

Gripping end portion 132 of inner shaft 130, proximal of branches 134, also includes a tapered outer surface 148 that is angled at an included angle ß that is approximately the same value as angle α, which, in an exemplary embodiment, may extend about 40 degrees.

Referring now to FIGS. 13-16, drive nut 150 is disposed over and coupled to handle end portion 135 of inner shaft 130. Drive nut 150 may be fixedly coupled to inner shaft 130 such as, for example, by welding drive nut 150 to inner shaft 130. Drive nut 150 includes a generally cylindrical body 152 that has a proximal end 154, a distal end 156, and a longitudinal axis 158 extending between proximal end 154 and distal end 156. When drive nut 150 is coupled to inner shaft 130 and inserted into outer body, as is shown in FIG. 15, longitudinal axis 158 is coaxial with longitudinal axis 106.

Distal end 156 includes an external thread 160 that engages internal thread 118 on outer body 110 to provide axial translation of outer body 110 with respect to inner shaft 130. Drive nut 150 also includes an annular portion 162 that is disposed between proximal end 154 and distal end 156. As described above, annular portion 162 has an outer diameter equal to the inner diameter of bore 119 of outer body 130 such that annular portion 162 may slide within and support proximal end 112 of outer body 110 as inner shaft 130 axially translates with respect to outer body 110.

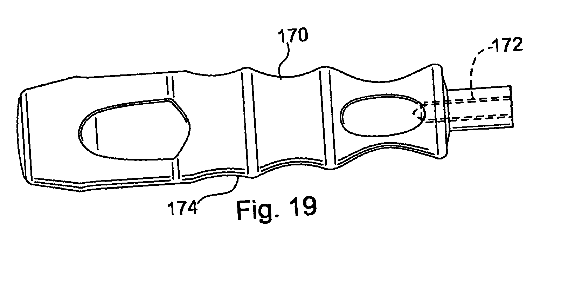

Referring to FIGS. 14 and 19, proximal end 154 of drive nut 150 includes a handle insert opening 164 into which an insert portion 172 of torque-applying handle 170 is inserted. Torque-applying handle 170 is fixedly coupled to drive nut 150, such as, for example, by welding so that, torque-applying handle 170 is fixedly coupled to inner shaft 130.

FIGS. 15-18 illustrate inner shaft 130 having been inserted through outer body 110. FIGS. 15 and 16 illustrate the cooperative engagement of internal thread 118 of outer body 110 with external thread 160 of drive nut 150. Outer body 110 is rotatable in a first direction to move gripping end portion 132 of inner shaft 130 proximally relative to outer body 110 and move branches 134 to the relatively closed, or gripping, position. Outer body 110 is also rotatable in a second direction to move gripping end portion 132 of inner shaft 130 distally relative to outer body 110 and move branches 134 to the relatively open, or release, position.

In FIGS. 15-18, outer body 110 and inner shaft 130 are disposed relative to each other such that counter-torque wrench 100 is in the gripping position, as shown in FIGS. 1 and 2. When counter-torque wrench 100 is moved to the gripping position, inner surface 120 of outer body 110, and particularly nub 122, engages outer surface 148 of inner shaft 130, compressing branches 134 toward longitudinal axis 106 such that branches 134 form or approximate a cylinder. In an exemplary embodiment, outer body 110 may be rotated about inner shaft 130 approximately 540 degrees (*i.e.* one and one-half rotations) in order to move from the gripping position of FIGS. 1 and 2 to the release position of FIGS. 3 and 4.

FIGS. 17 and 18 illustrate engagement of conically-shaped inner surface 120 of outer body 110 and tapered outer surface 148 of inner shaft 130 when counter-torque wrench 100 is in the gripping position. Nub 122 slidably engages inner shaft 130 to reduce the engagement area of inner shaft 130 with respect to outer body 110 in order to reduce the force required to disengage outer body 110 from inner shaft 130. In the exemplary embodiment, nub 122 is rounded as shown to reduce the amount of friction that must be overcome when disengaging outer body 110 from inner shaft 130.

As shown in FIG. 19, handle 170 includes a contoured gripping portion 174 that allows a user to grip the handle. Referring back to FIG. 5, handle 170 extends generally orthogonally relative to longitudinal axis 106 in order to allow a user to provide a counter-torque to the receiver body 52 and restrict or prevent movement of rod 70 as set screw 62 is being tightened into the receiver body.

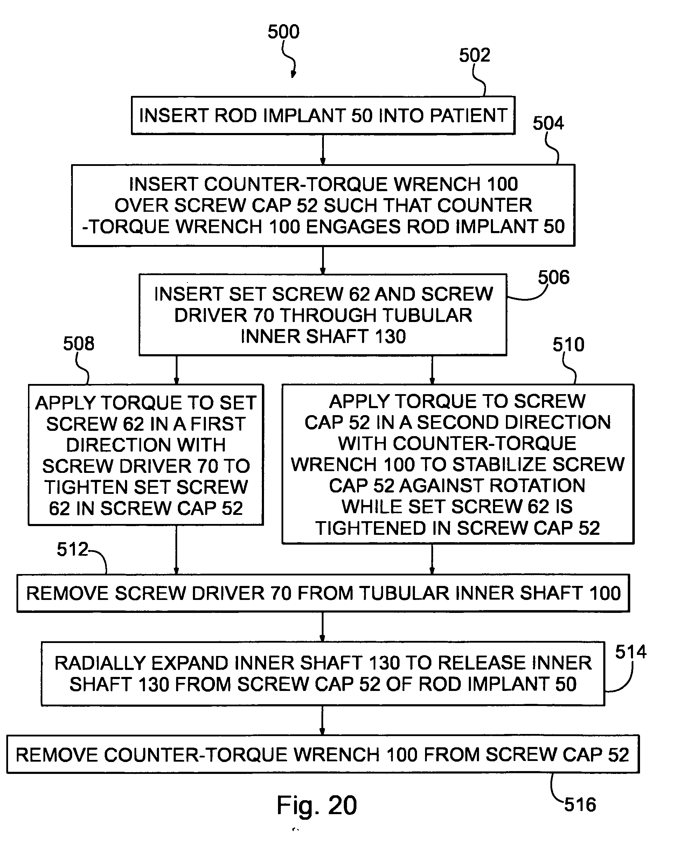

Referring now to FIGS. 1-5, as well as the flow chart 500 of FIG. 20, to use counter-torque wrench 100, in STEP 502, implant 50 is inserted into the patient (not shown). To fixate a rod 60 in implant 50, rod 60 is inserted into screw cap 52. In STEP 504, with counter-torque wrench 100 in the gripping position, counter-torque wrench 100 is inserted over screw cap 52, aligning diametrically opposed slots 136 on counter-torque wrench 100 with rod 60 such that counter-torque wrench 100 engages implant 50 and rod 60 is seated within slots 136. While STEP 504 describes counter-torque wrench 100 being inserted over screw cap 52 in the gripping position, those skilled in the art will recognize that counter-torque wrench 100 could also be inserted over screw cap 52 in the release position, and then moved to the gripping position prior to securing set screw 62 into screw cap 52.

Next, set screw 62 may be placed on a distal end of screw driver 70. In STEP 506, set screw 62 and screw driver 70 are inserted into handle end portion 135 of inner shaft 130 and through the length of inner shaft 130 toward distal end 132 of inner shaft 130 such that set screw 62 engages screw cap 52. Counter-torque wrench 100 is rotated slightly in a counter-clockwise direction when looking from proximal end 102 toward distal end 104 such that arcuate portion 138 engages rod 60 to restrict movement of rod 60 as set screw 62 is tightened. Alternatively, prior to STEP 504, set screw 62 may be inserted directly into screw cap 52 and initially threaded onto screw cap 52, with STEP 506 then including only the step of inserting the distal end of screw driver 70 through the length of inner shaft 130 until screw driver 70 engages set screw 62.

In STEP 508, torque is then applied with screw driver 70 to set screw 62 in a first direction such that set screw 62 is tightened in screw cap 52. Simultaneously, in STEP 510, a counter-torque is applied to screw cap 52 in a second direction, opposite the first direction, with counter-torque wrench 100 to stabilize screw cap 50 against rotation while set screw 62 is tightened in screw cap 52. Those skilled in the art will note that counter-torque may not be necessary during the entire course of tightening the set screw 62, and may only be needed in the latter stages of tightening (*e.g.*, the last turn of the screw driver) when torque on the set screw begins to transfer to the receiver body and the rod (*i.e.*, when the entire construct begins to rotate with the tightened set screw). In STEP 512, after set screw 62 is tightened, screw driver 70 is removed from inner shaft 130.

As set screw 62 is threaded into screw cap 52 in STEPS 508 and 510 above, screw cap 52 may have a tendency to expand, or "splay out." This expansion may cause screw cap 52 to impinge against the inside of branches 134 such that, when the user attempts to remove counter-torque wrench 100 from screw cap 52 after set screw 62 is tightened, counter-torque wrench 100 may stick to screw cap 52, making it difficult to remove counter-torque wrench 100.

In order to release counter-torque wrench 100 from screw cap 52, in STEP 514, gripping end portion 132 of inner shaft 130 may be radially expanded to release inner shaft 130 from screw cap 52. This is done by moving branches 134 out of engagement with each other by axially translating outer body 110 in a proximal direction relative to inner shaft 130, allowing branches 134 to disengage from each other, moving branches 134 from the gripping position to the release position. In STEP 516, after branches 134 have disengaged from each other, counter-torque wrench 100 may be removed from screw cap 52.

## Claims

1. A counter-torque wrench (100) for positioning and securing spinal fixation rods to screw and hook implants comprising:
a tubular outer body (110) having a distal end (114) and a proximal end (112); and
an inner shaft (130) disposed within the tubular outer body (110), the inner shaft (130) having a proximal end and a distal end (132) and being axially displaceable relative to the tubular outer body (110), the distal end of the inner shaft having a gripping end portion (132) disposed proximate to the distal end (114) of the tubular outer body (110), wherein the gripping end portion (132) comprises a plurality of branches (134) extending distally of the distal end (114) of the tubular outer body (110), the branches (134) being radially displaceable between a relatively open position, in which the branches (134) are displaced radially outwardly from one another, and a relatively closed position, in which the branches (134) are displaced radially inwardly toward one another, the distance between the branches (134) being greater in the open position than in the closed position, **characterized in that** the outer body (110) is rotatable in a first direction to move the gripping end portion (132) of the inner shaft (130) proximally relative to the outer body (110) and move the branches (134) to the relatively closed position, the outer body (110) being rotatable in a second direction to move the gripping end portion (132) of the inner shaft (130) distally relative to the outer body (110) and move the branches (134) to the relatively open position.

2. The counter-torque wrench according to claim 1, wherein the tubular outer body (110) includes an internal thread (118) and wherein the inner shaft (130) includes an external thread in cooperative engagement with the internal thread.

3. The counter-torque wrench according to any of the preceding claims, wherein the inner shaft (130) comprises a generally tubular body (131) extending between the gripping end portion (132) and the proximal end.

4. The counter-torque wrench according to any of the preceding claims, wherein the plurality of branches (134) comprises an even number of (134) branches.

5. The counter-torque wrench according to any of the preceding claims, further comprising a torque-applying handle (170) operatively connected to the tubular inner shaft (130).

6. The counter-torque wrench according to the preceding claims, wherein the outer body (110) is rotatable relative to the inner shaft (130) to move the inner shaft (130) relative to the outer body (110) between the relatively closed position and the relatively open position.

7. The counter-torque wrench according to any of claims 5 and 6, wherein the inner shaft (130) further comprises a handle end portion (135), and wherein the torque-applying handle (170) is fixedly coupled to the handle end portion (135).

8. The counter-torque wrench according to claim 7, wherein the inner shaft (130) is cannulated, forming a bore extending between the gripping end portion (132) and the handle end portion (135).

9. The counter-torque wrench according to any of the preceding claims, wherein the outer body (110) comprises a conically shaped inner surface (120).

10. The counter-torque wrench according to any of the preceding claims, wherein the gripping end portion (132) of the inner shaft (130) comprises a tapered outer surface (148) disposed proximally of the branches (134).

11. The counter-torque wrench according to claim 10, wherein the conically shaped inner surface (120) comprises a nub (122) extending radially inwardly in slidable engagement with the tapered outer surface (148) on the inner shaft (130).

12. The counter-torque wrench according to any of the preceding claims, wherein the plurality of branches (134) comprises a collet (133).

13. The counter-torque wrench according to any of the preceding claims, wherein the plurality of branches (134) comprises a pair of diametrically opposed cut outs for receiving a fixation rod.

## Patentansprüche

1. Gegendrehmomentschlüssel (100) zum Positionieren und Sichern von Wirbelsäulenfixationsstäben an Schrauben- und Hakenimplantaten, umfassend:
einen röhrenförmigen äußeren Körper (110) mit einem distalen Ende (114) und einem proximalen Ende (112); und
einen inneren Schaft (130), welcher innerhalb des röhrenförmigen äußeren Körpers (110) angeordnet ist, wobei der innere Schaft (130) ein proximales Ende und ein distales Ende (132) aufweist und relativ zu dem röhrenförmigen äußeren Körper (110) axial verschieblich ist, wobei das distale Ende des inneren Schafts einen Greifendebereich (132) aufweist, welcher nahe dem distalen Ende (114) des röhrenförmigen äußeren Körpers (110) angeordnet ist, wobei der Greifendebereich (132) eine Mehrzahl von Armen (134) aufweist, die sich distal des distalen Endes (114) des röhrenförmigen äußeren Körper (110) erstrecken, wobei die Arme (134) radial verschieblich sind zwischen einer relativ offenen Stellung, in der die Arme (134) radial auswärts voneinander weg verschoben sind, und einer relativ geschlossenen Stellung, in der die Arme (134) radial einwärts zueinander hin verschoben sind, wobei der Abstand zwischen den Armen (134) in der offenen Stellung größer ist als in der geschlossenen Stellung, **dadurch gekennzeichnet, dass** der äußere Körper (110) in einer ersten Richtung rotierbar ist, um den Greifendebereich (132) des inneren Schafts (130) relativ zu dem äußeren Körper (110) proximal zu bewegen und die Arme (134) in die relativ geschlossene Stellung zu bewegen, wobei der äußere Körper (110) in einer zweiten Richtung rotierbar ist, um den Greifendebereich (132) des inneren Schafts (130) relativ zu dem äußeren Körper (110) distal zu bewegen und die Arme (134) in die relativ offene Stellung zu bewegen.

2. Gegendrehmomentschlüssel nach Anspruch 1, wobei der röhrenförmige äußere Körper (110) ein Innengewinde (118) umfasst und wobei der innere Schaft (130) ein Außengewinde in zusammenwirkendem Eingriff mit dem Innengewinde umfasst.

3. Gegendrehmomentschlüssel nach einem der vorangehenden Ansprüche, wobei der innere Schaft (130) einen im Wesentlichen röhrenförmigen Körper (131) umfasst, der sich zwischen dem Greifendebereich (132) und dem proximalen Ende erstreckt.

4. Gegendrehmomentschlüssel nach einem der vorangehenden Ansprüche, wobei die Mehrzahl der Arme (134) eine gerade Anzahl von Armen (134) umfasst.

5. Gegendrehmomentschlüssel nach einem der vorangehenden Ansprüche, ferner umfassend einen drehmomentaufbringenden Griff (170), der mit dem röhrenförmigen inneren Schaft (130) wirkverbunden ist.

6. Gegendrehmomentschlüssel nach einem der vorangehenden Ansprüche, wobei der äußere Körper (110) relativ zu dem inneren Schaft (130) rotierbar ist, um den inneren Schaft (130) relativ zu dem äußeren Körper (110) zwischen der relativ geschlossenen Stellung und der relativ offenen Stellung zu bewegen.

7. Gegendrehmomentschlüssel nach einem der Ansprüche 5 und 6, wobei der innere Schaft (130) ferner einen Griffendebereich (135) umfasst und wobei der drehmomentaufbringende Griff (170) mit dem Griffendebereich (135) fest gekoppelt ist.

8. Gegendrehmomentschlüssel nach Anspruch 7, wobei der innere Schaft (130) kanüliert ist, so dass eine sich zwischen dem Greifendebereich (132) und dem Griffendebereich (135) erstreckende Bohrung gebildet ist.

9. Gegendrehmomentschlüssel nach einem der vorangehenden Ansprüche, wobei der äußere Körper (110) eine konisch geformte innere Oberfläche (120) aufweist.

10. Gegendrehmomentschlüssel nach einem der vorangehenden Ansprüche, wobei der Greifendebereich (132) des inneren Schafts (130) eine verjüngte äußere Oberfläche (148) aufweist, die proximal der Arme (134) angeordnet ist.

11. Gegendrehmomentschlüssel nach Anspruch 10, wobei die konisch geformte innere Oberfläche (120) eine Noppe (122) umfasst, die sich radial einwärts in gleitverschieblichem Eingriff mit der verjüngten äußeren Oberfläche (148) an dem inneren Schaft (130) erstreckt.

12. Gegendrehmomentschlüssel nach einem der vorangehenden Ansprüche, wobei die Mehrzahl von Armen (134) ein Spannfutter (133) umfasst.

13. Gegendrehmomentschlüssel nach einem der vorangehenden Ansprüche, wobei die Mehrzahl von Armen (134) ein Paar von diametral gegenüberliegenden Ausschnitten zur Aufnahme eines Fixationsstabes umfasst.

## Revendications

1. Clé à couple de réaction (100) pour positionner et immobiliser des tiges de fixation spinales pour visser et accrocher des implants, ladite clé à couple de réaction comprenant :
un corps extérieur tubulaire (110) ayant une extrémité distale (114) et une extrémité proximale (112) ; et
un arbre intérieur (130) disposé à l'intérieur du corps extérieur tubulaire (110), l'arbre intérieur (130) ayant une extrémité proximale et une extrémité distale (132) et étant déplaçable axialement par rapport au corps extérieur tubulaire (110), l'extrémité distale de l'arbre intérieur ayant une portion d'extrémité de prise (132) disposée près de l'extrémité distale (114) du corps extérieur tubulaire (110), la portion d'extrémité de prise (132) comprenant une pluralité de branches (134) s'étendant du côté distal de l'extrémité distale (114) du corps extérieur tubulaire (110), les branches (134) étant déplaçables radialement entre une position relativement ouverte, dans laquelle les branches (134) sont déplaçables radialement vers l'extérieur l'une par rapport à l'autre, et une position relativement fermée, dans laquelle les branches (134) sont déplacées radialement vers l'intérieur en direction l'une de l'autre, la distance entre les branches (134) étant supérieure dans la position ouverte à celle dans la position fermée, **caractérisée en ce que** le corps extérieur (110) est apte à tourner dans une première direction pour déplacer la portion d'extrémité de prise (132) de l'arbre intérieur (130) du côté proximal par rapport au corps extérieur (110) et déplacer les branches (134) vers la position relativement fermée, le corps extérieur (110) étant apte à tourner dans une seconde direction pour déplacer la portion d'extrémité de prise (132) de l'arbre intérieur (130) du côté distal par rapport au corps extérieur (110) et déplacer les branches (134) vers la position relativement ouverte.

2. Clé à couple de réaction selon la revendication 1, dans laquelle le corps extérieur tubulaire (110) inclut un filet interne (118) et dans lequel l'arbre intérieur (130) inclut un filet externe en prise avec le filet interne.

3. Clé à couple de réaction selon l'une quelconque des revendications précédentes, dans laquelle l'arbre intérieur (130) comprend un corps (131) de forme générale tubulaire s'étendant entre la portion d'extrémité de prise (132) et l'extrémité proximale.

4. Clé à couple de réaction selon l'une quelconque des revendications précédentes, dans laquelle la pluralité de branches (134) comprend un nombre pair de branches (134).

5. Clé à couple de réaction selon l'une quelconque des revendications précédentes, comprenant en outre une poignée dynamométrique (170) fonctionnellement raccordée à l'arbre intérieur tubulaire (130).

6. Clé à couple de réaction selon les revendications précédentes, dans laquelle le corps extérieur (110) est apte à tourner par rapport à l'arbre intérieur (130) pour déplacer l'arbre intérieur (130) par rapport au corps extérieur (110) entre la position relativement fermée et la position relativement ouverte.

7. Clé à couple de réaction selon l'une quelconque des revendications 5 et 6, dans laquelle l'arbre intérieur (130) comprend en outre une portion d'extrémité formant poignée (135), et dans laquelle la poignée dynamométrique (170) est couplée de façon fixe à la portion d'extrémité formant poignée (135).

8. Clé à couple de réaction selon la revendication 7, dans laquelle l'arbre intérieur (130) est cannelé, formant un perçage s'étendant entre la portion d'extrémité de prise (132) et la portion d'extrémité formant poignée (135).

9. Clé à couple de réaction selon l'une quelconque des revendications précédentes, dans laquelle le corps extérieur (110) comprend une surface intérieure (120) de forme conique.

10. Clé à couple de réaction selon l'une quelconque des revendications précédentes, dans laquelle la portion d'extrémité de prise (132) de l'arbre intérieur (130) comprend une surface extérieure conique (148) disposée du côté proximal des branches (134).

11. Clé à couple de réaction selon la revendication 10, dans laquelle la surface intérieure (120) de forme conique comprend une saillie (122) s'étendant radialement vers l'intérieur en engagement de coulissement avec la surface extérieure conique (148) sur l'arbre intérieur (130).

12. Clé à couple de réaction selon l'une quelconque des revendications précédentes, dans laquelle la pluralité de branches (134) comprend une douille (133).

13. Clé à couple de réaction selon l'une quelconque des revendications précédentes, dans laquelle la pluralité de branches (134) comprend une paire de découpes diamétralement opposées destinées à recevoir une barre de fixation.
